# EUROPEAN PATENT APPLICATION

(11) **EP 4 588 459 A1**
(43) Date of publication of application: **23.07.2025**
(21) Application number: 23884471.6
(22) Date of filing: 13.09.2023
(51) Int. Cl.: A61F 2/90

(54) **VASCULAR STENT**

(30) Priority: 31.10.2022 CN 202211342562
(71) Applicant: Shanghai MicroPort Medical (Group) Co., Ltd., Shanghai 201203 (CN)
(72) Inventor: GENG, Kangkang, Shanghai 201203 (CN); ZHOU, Qi, Shanghai 201203 (CN); HUANG, Haiyong, Shanghai 201203 (CN)
(74) Representative: Marks & Clerk LLP
(86) International application number: PCT/CN2023/118543
(87) International publication number: WO 2024/093539

(57) **Abstract**

The present application relates to a vascular stent, which includes a front stent section (10), a middle stent section (20) and a rear stent section (30) sequentially connected. An anchoring protrusion (50) is arranged on an outer surface of the front stent section (10) and/or the rear stent section (30). When the vascular stent is in the expanded state, the inner diameter of the middle stent section (20) is smaller than that of the front stent section (10) and the rear stent section (30). The front stent section (10) is configured to contact an inner wall of a blood vessel (40), the rear stent section (30) is configured to contact the inner wall of the blood vessel (40), and the anchoring protrusion (50) is configured to anchor the vascular stent. That is, the anchoring protrusion (50) is attached to the inner wall of the blood vessel (40) and inserted into the blood vessel (40) for anchoring.

## Description

### CROSS-REFERENCES TO RELATED APPLICATIONS

This application claims priority to Chinese patent application No. 2022113425625, filed on October 31, 2022, and entitled "VASCULAR STENT", the content of which is hereby incorporated in its entirety by reference.

### TECHNICAL FIELD

The present application relates to the technical field of medical devices, and more particularly, to a vascular stent.

### BACKGROUND

Angina pectoris is a clinical syndrome caused by acute and transient ischemia and hypoxia of the myocardium due to insufficient coronary blood supply. The characteristic of an attack is paroxysmal chest pressure-like pain, primarily located behind the sternum. The pain may radiate to the precordial area and the left upper limb. Typically, anti-anginal medications, percutaneous coronary intervention (PCI), or coronary artery bypass grafting (CABG) can improve angina symptoms. However, even after receiving these treatments, 2%-24% of coronary heart disease patients experience angina attacks daily or weekly. This condition is referred to as refractory angina, which specifically refers to angina that recurs for more than 3 months due to reversible local ischemia, and cannot be controlled by medications, CABG, or PCI. Improving angina is crucial for enhancing the patient's quality of life.

In related art, using implantable stents for coronary venous sinus vessels is one of the effective methods for treating refractory angina. The diameter of the implantable stent gradually increases from the middle to both ends and ultimately stabilizes, forming an expanded end with a large caliber and a fixed diameter at both ends. After stent implantation, blood flows through the lumen of the device. When passing through the smaller-diameter middle region, the blood flow speed increases due to the reduction in the cross-sectional area of the flow path, generating a pressure gradient across the device, which leads to the redistribution of blood from the epicardium to the endocardium. This helps reduce endocardial ischemia, ultimately reducing the occurrence of angina.

However, the anchorage of this implantable stent in the vessel mainly depends on the contact area and fit between the expanded ends and the vascular wall tissue. When the length of the expanded end is short, the support is not secure, and the implantable stent may displace or even fall off. When the length of the expanded end is long, the stability of the vascular stent after release is better. However, it causes the vessel wall to become irregular, which can lead to thrombosis within the vascular stent. Additionally, it alters the physiological curvature and flexibility of the vessel, and prolonged rigid restraint is unfavorable for the subsequent restoration of vascular function.

### SUMMARY

According to various embodiments of the present application, the present application provides a vascular stent.

The technical solution is as follows. A vascular stent is provided, including:
a front stent section, a middle stent section, and a rear stent section connected sequentially, an outer surface of the front stent section and/or the rear stent section being provided with an anchoring protrusion.

When the vascular stent is expanded, an internal diameter of the middle stent section is smaller than internal diameters of the front stent section and the rear stent section, the front stent section is configured to contact an inner wall of a blood vessel, the rear stent section is configured to contact the inner wall of the blood vessel, and the anchoring protrusion is configured to anchor the vascular stent.

In one of the embodiments, a proximal end and/or a distal end of the front stent section is provided with a radiopaque marker, and a proximal end and/or a distal end of the rear stent section is provided with a radiopaque marker.

In one of the embodiments, the front stent section, the middle stent section, the rear stent section, and the anchoring protrusion are integrally formed from a shape memory alloy material.

In one of the embodiments, the front stent section, the middle stent section and the rear stent section are integrally formed from a shape memory alloy material to obtain a main body of the vascular stent, the anchoring protrusion is connected with the main body of the vascular stent by clamping, welding or bonding, and the anchoring protrusion is made of a degradable material.

In one of the embodiments, the front stent section and/or the rear stent section includes a connecting portion provided with the anchoring protrusion.

In one of the embodiments, an outer surface of the front stent section and/or the rear stent section carries a drug.

In one of the embodiments, the front stent section, the middle stent section, and the rear stent section each have a mesh structure, shapes of mesh apertures of the front stent section, the middle stent section, and the rear stent section are each rectangular, rhombic, circular, or oval, and an area of the mesh aperture of the middle stent section gradually decreases from both ends of the middle stent section towards a middle region of the middle stent section.

In one of the embodiments, the front stent section, the middle stent section, and the rear stent section of the stent each include multiple unit frames forming the mesh apertures, each unit frame includes at least four connecting bars sequentially connected end to end and connecting portions each connected between adjacent connecting bars, and two adjacent unit frames share one connecting portion.

In one of the embodiments, an outer surface and/or an inner surface of the middle stent section is provided with a covering.

In one of the embodiments, a minimum inner diameter of the middle stent section in an expanded state is defined as D1, where D1 ranges from 1.5mm to 6mm; an inner diameter of the front stent section in an expanded state is defined as D2, where D2 ranges from 3mm to 18mm; an inner diameter of the rear stent section in an expanded state is defined as D3, where D3 ranges from 3mm to 18mm; and an inner diameter of the middle stent section gradually decreases from both two ends of the middle stent section the to a center of the middle stent section.

In one of the embodiments, a length of the front stent section in an expanded state is defined as L1, and a length of the rear stent section in an expanded state is defined as L2, where L1 ranges from 0.2mm to 15mm, and L2 ranges from 0.2mm to 15mm.

Details of one or more embodiments of the present application are presented in the following drawings and descriptions. Other features, purposes and advantages of this application will be evident from the description, drawings and claims.

### BRIEF DESCRIPTION OF THE DRAWINGS

The drawings that form part of this application are used to provide further understanding of the present application. The illustrative embodiments and their descriptions are provided to explain the application and do not constitute improper limitations to the application.

To more clearly illustrate the technical solutions in the embodiments of the present application, a brief introduction to the drawings needed in the description of the embodiments will be provided below. Clearly, the drawings described below are merely some embodiments of the present application, and those skilled in the art may derive other drawings from these without any inventive effort.
FIG. 1 is a schematic structural diagram of a vascular stent according to an embodiment of the present application.
FIG. 2 is a schematic structural diagram of a delivery device entering into the interior of a blood vessel according to an embodiment of the present application.
FIG. 3 is a schematic structural diagram of a delivery device releasing an anterior section of a vascular stent according to an embodiment of the present application.
FIG. 4 is a schematic diagram of a delivery device being withdrawn outward after completely releasing a vascular stent according to an embodiment of the present application.
FIG. 5 is a schematic structural diagram of a vascular stent implanted into a blood vessel according to an embodiment of the present application.
FIG. 6 is a schematic structural diagram of a vascular stent on which an anchoring protrusion is arranged according to an embodiment of the present application.
FIG. 7 is a schematic structural diagram of a vascular stent on which an anchoring protrusion is arranged according to another embodiment of the present application.
FIG. 8 is a schematic structural diagram of a vascular stent on which an anchoring protrusion is arranged according to yet another embodiment of the present application.
FIG. 9 is a side view of the structure shown in FIG. 1.
FIG. 10 is a schematic structural diagram of a vascular stent after being cut and unfolded according to an embodiment of the present application.
FIG. 11 is a schematic structural diagram of a vascular stent on which a covering is arranged according to an embodiment of the present application.
FIG. 12 is a schematic structural diagram of a vascular stent according to another embodiment of the present application.
FIG. 13 is a schematic structural diagram of a vascular stent after being cut and unfolded according to another embodiment of the present application.
FIG. 14 is a schematic structural diagram of a vascular stent after being cut and unfolded according to yet another embodiment of the present application.
10. front stent section; 20. middle stent section; 30. rear stent section; 40. blood vessel; 50. anchoring protrusion; 60. radiopaque marker; 70. mesh aperture; 80. unit frame; 81. connecting bar; 82. connecting portion; 91. covering; 92. plaque; 93. delivery device.

### DETAILED DESCRIPTION OF THE EMBODIMENTS

In order to make the above purposes, characteristics and advantages of the present application more obvious and easier to understand, the specific implementation of the present application is explained in detail in combination with the attached drawings below. A number of specific details are set out in the following description to facilitate a full understanding of the application. However, the present application can be implemented in a manner different from others described herein, and a person skilled in the art can make similar improvements without violating the content of the present application, so that the present application is not limited by the specific embodiments disclosed below.

In the present application, the terms "distal end" and "proximal end" refer to the relative orientation, relative position, and direction of each other's elements or actions from the perspective of the doctor using the medical device. Although "distal end" and "proximal end" are not restrictive, "distal end" generally refers to the end that first enters the patient's body, and the opposite end is "proximal end", in other words, "distal end" is more distant from the doctor than "proximal end".

The vascular stent in this embodiment can be suitable for use in the coronary sinus, other coronary veins, or other veins with non-muscular walls, as well as in arteries.

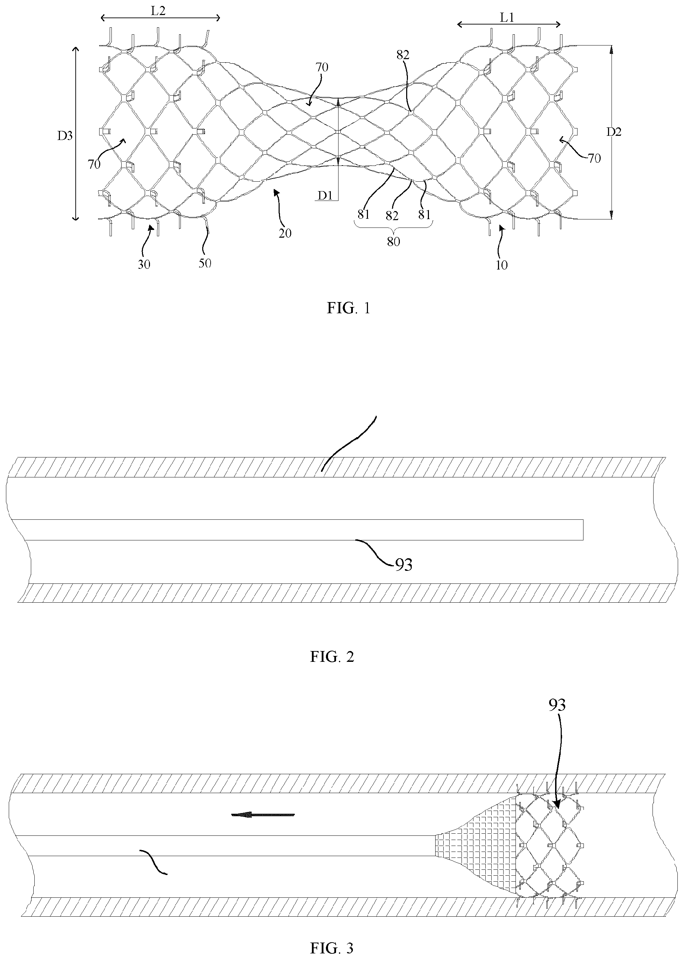

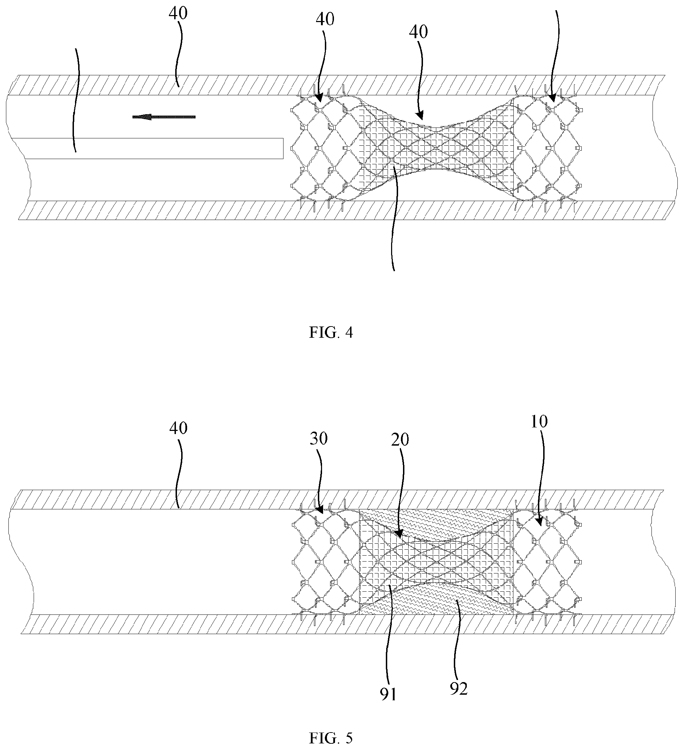

Referring to FIGS. 1-5, FIG. 1 shows a schematic diagram of the structure of a vascular stent according to an embodiment of the present application, and FIG. 2 shows a schematic diagram of a delivery device 93 entering into the interior of a blood vessel 40 according to an embodiment of the present application, FIG. 3 shows a schematic diagram of a delivery device 93 releasing a front stent section 10 according to an embodiment of the present application, FIG. 4 shows a schematic diagram of a delivery device 93 being withdrawn outward after completely releasing the vascular stent according to an embodiment of the present application, and FIG. 5 shows a schematic diagram of a vascular stent implanted into a blood vessel 40 according to an embodiment of the present application. An embodiment of the present application provides a vascular stent, which has the advantages of more secure fixation and accurate positioning. The vascular stent includes a front stent section 10, a middle stent section 20, and a rear stent section 30, which are connected sequentially. An outer surface of the front stent section 10 and/or the rear stent section 30 is provided with an anchoring protrusion 50. When the vascular stent is in an expanded state, the internal diameter of the middle stent section 20 is smaller than the internal diameters of the front stent section 10 and the rear stent section 30. The front stent section 10 is configured to contact an inner wall of a blood vessel 40, the rear stent section 30 is configured to contact the inner wall of the blood vessel 40, and the anchoring protrusion 50 is configured to anchor the vascular stent, that is, the anchoring protrusion 50 is attached to the inner wall of the blood vessel 40 and inserted into the blood vessel 40 for anchoring.

When the above-mentioned vascular stent is implanted into a target position inside the blood vessel 40 through the delivery device 93, the vascular stent is in an expanded state, i.e., an expanded configuration. The front stent section 10 and the rear stent section 30 both make contact with the inner wall of the vessel 40. That is, once expanded, the vascular stent forms portions that contact the inner wall of the vessel 40 and have a certain length, ensuring that the contact area between the vascular stent and the inner wall of the vessel 40 is sufficiently large. This provides the vascular stent with anchoring effect and support, reducing the risk of displacement and detachment of the vascular stent. Moreover, the anchoring protrusion 50 on the front stent section 10 and/or the rear stent section 30 is pierced into the wall of the blood vessel 40 to prevent the vascular stent from shifting or detaching, so that the support and fixation of the vascular stent is more stable and secure.

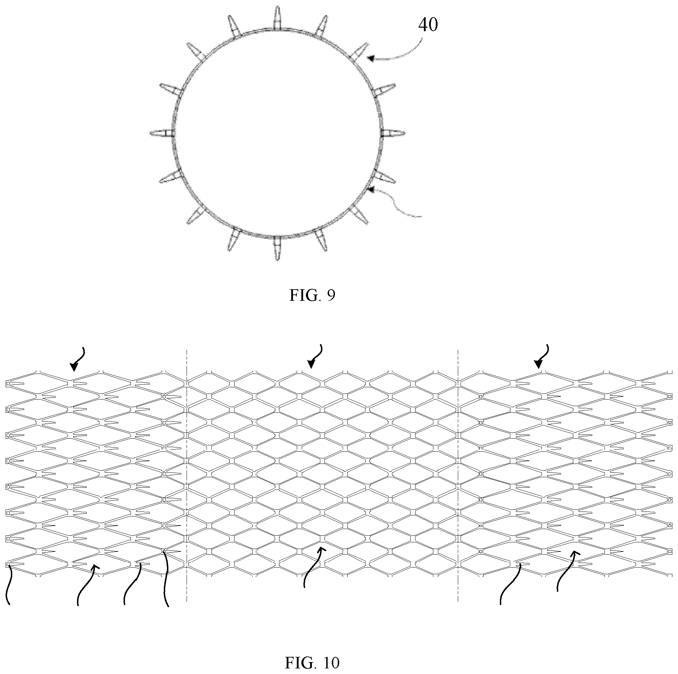

Referring to FIG. 10, FIG. 10 shows a schematic diagram of a vascular stent after being cut and unfolded according to an embodiment of the present application. It should be noted that the cut and unfolded structure shown in FIG. 10 is a semi-finished product that has not undergone shaping treatment or balloon-expansion treatment, while an expanded structure of a finished product is as shown in FIG. 1. In an embodiment, a proximal end and/or a distal end of the front stent section 10 is provided with a radiopaque marker 60. A proximal end and/or a distal end of the rear stent section 30 is provided with a radiopaque marker 60. In this way, during the implantation of the vascular stent, the fit of the front stent section10 and the rear stent section30 with the wall of the blood vessel 40 can be clearly monitored through the radiopaque markers 60, allowing for real-time positioning, adjustment, and precise deployment of the vascular stent at the implantation position and ensuring that the vascular stent better conforms to the anatomical structure of a coronary sinus.

Referring to FIG. 10, in an embodiment, when the vascular stent is in a fully expanded state, a radiopaque marker 60 is provided at the proximal end of the front stent section 10, or a plurality of radiopaque markers 60 are provided, for example, evenly along the circumferential direction at the proximal end. A radiopaque marker 60 is provided at the distal end of the front stent section 10 or a plurality of radiopaque markers 60 are provided, for example, evenly along the circumferential direction of the distal end. In addition, similarly, a radiopaque marker 60 is provided at the proximal end of the rear stent section 30, or a plurality of radiopaque markers 60 are provided, for example, evenly along the circumferential direction of the proximal end. A radiopaque marker 60 is provided at the distal end of the rear stent section 30 or a plurality of radiopaque markers 60 are provided, for example, evenly along the circumferential direction of the distal end.

In an embodiment, a middle part or other parts of the front stent section 10 may also be provided with one or more radiopaque markers 60 according to actual needs. The middle part or other parts of the rear stent section 30 may also be provided with one or more radiopaque markers 60 according to actual needs.

Alternatively, the material of the radiopaque marker 60 is a radiopaque substance, which includes any suitable material such as gold, tantalum, platinum, tungsten, barium sulfate, zirconia, or a combination thereof, or biodegradable radiopaque polymer materials. Additionally, the shape of the radiopaque marker 60 can be flexibly adjusted and configured according to practical needs, and is not limited here, including but not limited to regular and irregular shapes such as dots, circles, stars, triangles, squares, and so on.

In an embodiment, the front stent section 10, the middle stent section 20, the rear stent section 30, and the anchoring protrusion 50 are integrally formed from a shape memory alloy material.

Specifically, shape memory alloy materials include, but are not limited to, 316L stainless steel, platinum-iridium, platinum-chromium, cobalt-chromium, platinum-tungsten, nickel-titanium, or other metals or alloys, and various combinations thereof.

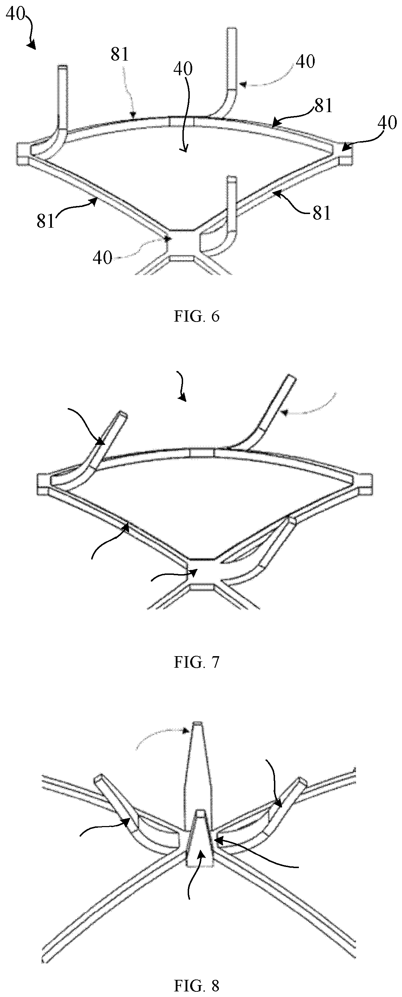

In a specific embodiment, a semi-finished vascular stent is obtained through cutting or weaving forming, as shown in FIG. 10. When the cutting is completed to obtain the semi-finished vascular stent, the anchoring protrusion 50 as a whole is parallel to the axis of the vascular stent. Then, the finished product is obtained by heat treatment and shaping of the semi-finished product through relevant molds, as shown in FIG. 1. Each anchoring protrusion 50 includes a horizontal section parallel to the axis of the vascular stent (i.e., unbent), and a curved section and an extended section that are formed by bending and extending radially outward. The extension direction of the extended section forms an angle with the axial direction of the vascular stent, for example, an angle of 30° to 90°. The curved section is smoothly connected to the extended section through a small fillet. Referring to FIG. 6 to 8, FIG. 6 illustrates a schematic structural diagram of a vascular stent on which anchoring protrusions 50 are arranged according to an embodiment of the present application, FIG. 7 illustrates a schematic structural diagram of a vascular stent on which anchoring protrusions 50 are arranged according to another embodiment of the present application, and FIG. 8 illustrates a schematic structural diagram of a vascular stent on which anchoring protrusions 50 are arranged in yet another embodiment of the present application. The extension direction of the extended section of the anchoring protrusion 50 shown in FIG. 6 is perpendicular to the axial direction of the vascular stent. The extension direction of the extended section of the anchoring protrusion 50 shown in FIG. 7 is at 45° to the axial direction of the vascular stent. As shown in FIG. 8, four anchoring protrusions 50 are connected to a same connecting portion 82, and are oriented in different directions and evenly arranged along the circumferential direction of the connecting portion 82.

In another embodiment, the front stent section 10, the middle stent section 20, and the rear stent section 30 are integrally formed from a shape memory alloy material to obtain a main body of the vascular stent. The anchoring protrusion 50 is connected with the main body of the vascular stent by clamping, welding or bonding. The anchoring protrusion 50 is made of a degradable material. In this way, the anchoring protrusion 50 plays a fixing role in the early stage of the implantation of the vascular stent into the blood vessel 40, preventing the vascular stent from shifting or detaching. After, for example, several months, when intimal hyperplasia stabilizes the vascular stent inside the vessel 40, the anchoring protrusion 50 will be completely absorbed or degraded without residue, which reduces the amount of permanent material and minimizes the adverse impact of the vascular stent on the vessel 40.

In an embodiment, the anchoring protrusion 50 is made of an absorbable metal material, absorbable polymer material and its copolymers, or a combination thereof. Specifically, the absorbable metal material includes one or more of magnesium-based, iron-based, or zinc-based alloys. The absorbable polymer material and its copolymer includes one or more of polylactic acid, polycaprolactone, polycarbonate, polylactide, polyglycolide, polycyanoacrylate, polycaprolactone, polyorthoester, polyphosphazene, or polyglycolic acid.

Alternatively, the anchoring protrusion 50 includes, but is not limited to, miniature barbs, sharp components, hooks, plant-like thorns, and other similar structures, and can be flexibly adjusted into various shapes according to actual needs. In addition, the length of each anchoring protrusion 50 does not exceed the width of the vessel 40. Moreover, the anchoring protrusions 50 may have the same or different structures, and the same or different lengths.

Alternatively, the number of the anchoring protrusions 50 is not limited and can vary depending on factors such as the target implantation site and the size of the vascular stent.

Alternatively, the anchoring protrusions 50 can be distributed in partial areas of the front stent section 10 and the rear stent section 30, such as a proximal edge, a distal edge, or a central region, and are evenly distributed along at least one circumference after the vascular stent is expanded. It can be understood that the anchoring protrusions 50 can also be distributed across all areas of the front stent section 10 and rear stent section 30.

Referring to FIGS. 1-9, FIG. 9 shows a side view of the structure shown in FIG. 1. In an embodiment, each connecting portion 82 of the front stent section 10 is provided with anchoring protrusions 50, and each connecting portion 82 of the rear stent section 30 is also provided with anchoring protrusions 50. In this way, the displacement or detachment of the vascular stent can be prevented, ensuring a more stable and secure support and fixation of the vascular stent.

Alternatively, the anchoring protrusions 50 are arranged only at the edge regions of both ends of the vascular stent. This configuration reduces the number of anchoring protrusions 50, thereby minimizing the damage to the vessel 40.

In an embodiment, the outer surface of the front stent section 10 and/or the rear stent section 30 carries a drug. The types of the drug are not limited in this application, and may include, but are not limited to, drugs that promote tissue growth. In an embodiment, the drug may include an antithrombotic agent, an anticoagulant, an antiplatelet agent, an antitumor agent, an antiproliferative agent, an antibiotic, an anti-inflammatory agent, a gene therapy agent, recombinant DNA products, recombinant RNA products, collagen, collagen derivatives, protein analogs, sugars, sugar derivatives, smooth muscle cell proliferation inhibitors, endothelial cell migration, proliferation, and/or survival promoters, or a combination thereof. When the drug specifically promotes tissue growth, it can facilitate the growth of the intima, thereby ensuring the long-term stability of the vascular stent after implantation into the vessel 40.

In an embodiment, the front stent section 10, the middle stent section 20, and the rear stent section 30 are all mesh structures.

Alternatively, the shape of mesh apertures 70 in the front stent section 10, middle stent section 20, and rear stent section 30 is rectangular, diamond-shaped, circular, elliptical, or other shapes, or various combinations thereof.

Referring to FIGS. 1-10, alternatively, the area of the mesh apertures 70 of the middle stent section 20 gradually decreases from both ends of the middle stent section 20 towards the middle region of the middle stent section 20. In this way, the blood flow through the sidewall of the middle stent section 20 be restricted (the viscosity of the blood hinders the passage through the mesh apertures 70 at the size-reduced middle region), and it can also prevent substances such as thrombus from entering the interior of the vascular stent through the mesh apertures 70 at the narrowed region.

Additionally, the area of the mesh apertures 70 in the front stent section 10 and the rear stent section 30 is larger than that in the middle stent section 20. In the same circumferential direction, the numbers of mesh apertures 70 in the front stent section 10, the rear stent section 30, and the middle stent section 20 are the same, ensuring that the front stent section 10 and the rear stent section 30 have a relatively larger expansion diameter when expanded.

As an alternative solution, the front stent section 10, middle stent section 20, and rear stent section 30 can each be a waveform shape. In this case, it can be understood that the mesh apertures 70 in the front stent section 10, middle stent section 20, and rear stent section 30 are not regular shapes like rectangles, diamonds, circles, or ellipses, but are irregular-shaped apertures, such as elongate holes, specifically, waist-shaped holes, strip-shaped holes, and so on.

Referring to FIGS. 1-6, in an embodiment, the front stent section 10, middle stent section 20, and rear stent section 30 each include unit frames 80 forming the mesh apertures 70. There are multiple unit frames 80, with each unit frame 80 including at least four connecting bars 81 that are sequentially connected end to end and connecting portions 82 that each connect two adjacent connecting bars 81. Two adjacent unit frames share a connecting portion 82.

The size of the mesh apertures 70 in the front stent section 10, middle stent section 20, and rear stent section 30 can be flexibly adjusted and configured according to actual requirements. Optionally, the size of the mesh apertures 70 in the front stent section 10 and rear stent section 30 is larger than that in the middle stent section 20, i.e., the size of the unit frames 80 in the front stent section 10 and rear stent section 30 is larger than that in the middle stent section 20. Additionally, the size of the unit frame 80 in the front stent section 10 can be the same as, or different from, the size of the unit frame 80 in the rear stent section 30.

Additionally, the connecting portion 82 can be, for example, rectangular, diamond-shaped, circular, elliptical, or other shapes. Furthermore, the connecting bars 81 and the connecting portion 82 are smoothly connected through an arc, which helps alleviate stress concentration during expansion.

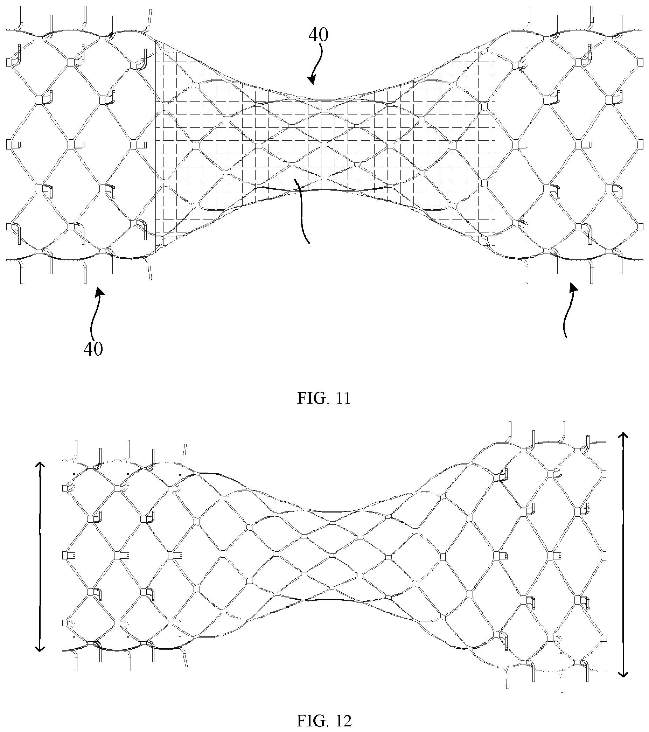

Referring to FIG. 11, FIG. 11 is a schematic diagram of a vascular stent with a covering 91 in an embodiment of the present application. In an embodiment, the outer and/or inner surface of the middle stent section 20 is provided with a covering 91. In this way, the covering 91 can restrict blood flow through the surface mesh apertures 70 of the middle stent section 20 or turbulent blood flow at the narrowed region of the middle stent section 20, which can reduce the risk of embolism transfer. After the vascular stent with the covering 91 is implanted, it can quickly establish a pressure gradient for blood flow, thereby immediately alleviating angina.

Furthermore, the covering 91 can be any material made of fabric or synthetic materials such as polymers, or the covering 91 can be any tissue, such as pericardial tissue or other biological tissues, or it can be made of a biodegradable material. Optionally, the covering 91 is attached to the middle stent section 20 through suturing, heat sealing, or embossing.

As an alternative, the surface of the vascular stent may not have a covering 91.

In the present embodiment, the implantation of the vascular stent is, for example, balloon dilation. The vascular stent, together with the delivery device 93, is passed through the jugular vein or subclavian vein, via the superior vena cava and/or femoral vein, inferior vena cava, and is inserted into the right atrium of the heart. Once entering the right atrium, the vascular stent is guided into, for example, the coronary sinus blood vessel 40. The balloon is then inflated to expand and deploy the vascular stent until the front stent section 10 and rear stent section 30 are fully in contact with the wall of the vessel 40, after which the balloon is deflated and withdrawn. At this point, the proximal end of the vascular stent is located at a distance of, for example, 1cm-8cm from the ostium of the right atrium, specifically, for example, 2cm-4cm.

Optionally, after expansion, the front stent section 10 and rear stent section 30 each have a constant internal diameter, and the internal diameters can either be the same or different. Alternatively, after expansion, the front stent section 10 and rear stent section 30 may form conical sections with gradually changing internal diameters.

It should be noted that the internal diameters of the front stent section 10, middle stent section 20, and rear stent section 30 when expanded can be flexibly adjusted and configured according to actual needs, as long as they ensure that the front stent section 10 and rear stent section 30, when expanded, make contact with the inner wall of the vessel 40 to provide support and fixation.

In an embodiment, the minimum internal diameter of the middle stent section 20 in the expanded state is defined as D1, and D1 is equal to, but is not limited to, 1.5mm-6mm. Studies have found that when configured within this range, as blood flows into the middle stent section 20, the cross-sectional area of the flow path decreases, which accelerates the blood flow speed and creates a pressure gradient, redistributing the blood to the areas of the myocardium that need blood most.

In an embodiment, the internal diameter of the front stent section 10 in the expanded state is defined as D2, where D2 is equal to, but is not limited to, 3mm-18mm. The internal diameter of the rear stent section 30 in the expanded state is defined as D3, where D3 is equal to, but is not limited to, 3mm-18mm. Therefore, when the front stent section 10 and the rear stent section 30 are implanted into the blood vessel 40, the front stent section 10 and rear stent section 30, when expanded, will come into contact with the inner wall of the vessel 40 to provide support and fixation.

The internal diameters of the front stent section 10 and the rear stent section 30 can be the same or different, and each can be configured as a section with a constant internal diameter or a varying internal diameter.

Referring to FIG. 1, in an embodiment, when expanded, the front stent section 10 and rear stent section 30 are sections with a constant internal diameter.

In another embodiment, the front stent section 10 and rear stent section 30 are conical sections after expansion, with an internal diameter that gradually changes to accommodate blood vessels 40 with significant variations in internal diameter. Specifically, after expansion, the internal diameters of the front stent section 10 and rear stent section 30 each gradually increase, gradually decrease, first gradually increase and then gradually decrease, or first gradually decrease and then gradually increase in the direction from one end to the other.

Referring to FIG. 12, FIG. 12 schematically illustrates the structure of the vascular stent in another embodiment of the present application. The difference between the structure shown in FIG. 12 and that in FIG. 1 is that the internal diameter D2 of the front stent section 10 in the expanded state is different from the internal diameter D3 of the rear stent section 30 in the expanded state. Optionally, in FIG. 12, the internal diameter D2 of the front stent section 10 in the expanded state is greater than the internal diameter D3 of the rear stent section 30 in the expanded state.

Referring to FIG. 1 or FIG. 12, optionally, the internal diameter of the middle stent section 20 gradually decreases from both ends toward the center, and the profile of the middle stent section 20 is streamlined. After the vascular stent is implanted into the blood vessel 40, as blood flow is directed and intimal hyperplasia occurs, the narrowed passage of the middle stent section 20 becomes the primary passage for blood reflux, thereby changing the pressure gradient upstream, which leads to the redistribution of blood from the epicardium to the endocardium, helping to reduce myocardial ischemia and ultimately decrease the occurrence of angina. In addition, the gap between the middle stent section 20 and the inner wall of the blood vessel 40 will be filled with the proliferated intima or stable plaques 92 (as shown in FIG. 5), which helps prevent the vascular stent from shifting or detaching, improving the stability of the vascular stent within the blood vessel 40. The minimum internal diameter D1 of the middle stent section 20 when expanded is set to be between 1.5mm and 6mm. Optionally, D1 may be specifically 1.5mm, 2mm, 3mm, 4mm, 5mm, or 6mm.

When the main body of the vascular stent is in the expanded state, the front stent section 10 and the rear stent section 30 are presented as sections with a certain length and a large internal diameter, which are in contact with the wall of the blood vessel 40. The lengths of these sections are denoted as L1 and L2, respectively.

In an embodiment, the length of the front stent section 10 can either be the same as the length of the rear stent section 30, or differ from the length of the rear stent section 30. The length can be flexibly adjusted and set according to actual needs, and no specific limitations are placed here.

Referring to FIG. 1, in an embodiment, the length of the front stent section 10 in the expanded state is defined as L1, and the length of the rear stent section 30 in the expanded state is defined as L2. Specifically, L1 is in the range of 0.2mm to 15mm, and L2 is in the range of 0.2mm to 15mm.

In a specific embodiment, L1 includes, but is not limited to, a range of 0.5mm to 5mm, and L2 includes, but is not limited to, a range of 0.5mm to 5mm. These values can also be set to any value smaller than 0.5mm or greater than 5mm based on actual needs.

The length of the front stent section 10 is greater than the length of the rear stent section 30. The front stent section 10 is considered as the distal section when the vascular stent is released, and the length of the front stent section 10 is not smaller than the length of the rear stent section 30. The larger the internal diameter of the coronary sinus, the longer the front stent section 10 is designed to be, so as to fix the vascular stent at a head position first.

Additionally, the rear stent section 30 is regarded as the proximal section when released. Optionally, after the vascular stent is released inside the vessel 40, the distance between the proximal end of the rear stent section 30 and the ostium of the right atrium can be controlled to be, for example, 1cm to 8cm, specifically 2cm to 4cm.

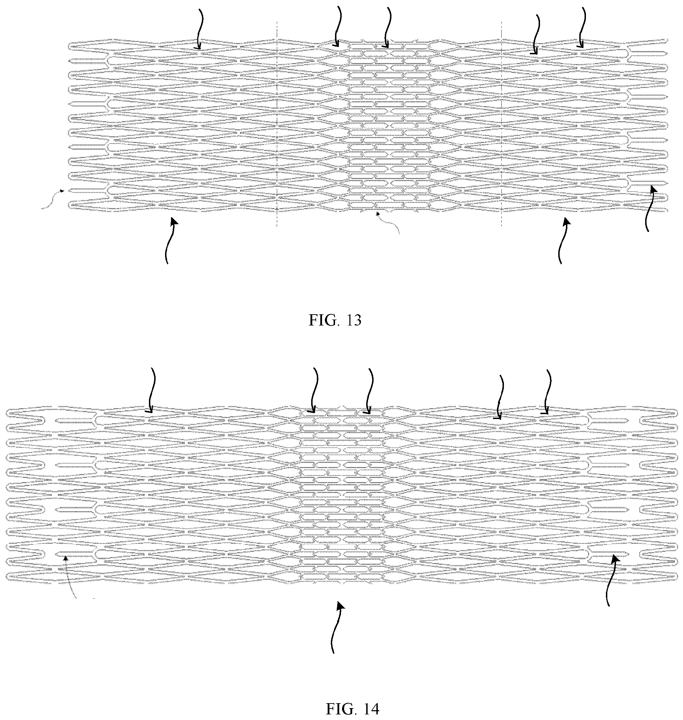

As some optional solutions, referring to FIG. 13 and FIG. 14, FIG. 13 shows a schematic diagram of the structure of the vascular stent after it is cut and unfolded according to another embodiment of the present application, and FIG. 14 shows a schematic diagram of the structure of the vascular stent after it is cut and unfolded according to yet another embodiment of the present application. It should be noted that the cut and unfolded structure shown in FIG. 13 and FIG. 14 is a semi-finished product that has not undergone shaping or balloon dilation treatments. The anchoring protrusions 50 are only located at the edge regions of both ends of the vascular stent, and the number of anchoring protrusions 50 in this configuration is relatively reduced, thereby minimizing damage to the vessel 40. Additionally, there is no covering 91 on the main body of the vascular stent. The main body of the vascular stent is a support structure with mesh apertures 70, with the area of the mesh aperture 70 gradually decreasing from both ends toward the middle region of the main body of the vascular stent. The mesh apertures 70 of the middle stent section 20 are denser, which helps reduce the blood flow passing through the side walls of the vascular stent and prevents thrombotic substances from passing through the vascular stent, thereby preventing embolism.

The technical features of the above embodiments can be combined in any way. For the sake of brevity, not all possible combinations of the technical features in the above embodiments have been described. However, as long as the combinations of these technical features do not conflict, they should be considered within the scope disclosed in this specification.

The embodiments described above only represent several possible implementations of this application, and are described in a more specific and detailed manner, but should not be understood as limiting the scope of the patent application. It should be noted that, for a person skilled in the art, many modifications and improvements can be made without departing from the concept of this application, and these are all within the scope of protection of this application. Therefore, the scope of protection of the patent application should be determined by the appended claims.

In the description of this application, it should be understood that terms such as "center", "longitudinal", "lateral", "length", "width", "thickness", "up", "down", "front", "rear", "left", "right", "vertical", "horizontal", "top", "bottom", "inner", "outer", "clockwise", "counterclockwise", "axial", "radial", and "circumferential" refer to the orientation or positional relationships based on the orientation or positional relationships shown in the accompanying drawings. These are used only for the convenience of describing and simplifying the description of the application, and do not indicate or imply that the devices or components must have a specific orientation or be constructed and operated in a specific orientation, and should not be understood as a limitation on the application.

In addition, the terms "first" and "second" are used only for descriptive purposes, and should not be understood as indicating or implying relative importance or implicitly indicating the number of features. Therefore, features with "first" and "second" can explicitly or implicitly include at least one of the features in question. In this application, "a plurality of" means at least two, such as two, three, etc., unless otherwise clearly specified.

In this application, unless explicitly stated otherwise, terms such as "install", "connected", "coupled", "fixed", etc., should be broadly understood. For example, they could refer to fixed connections or detachable connections, or integration; they could be mechanical connections or electrical connections; they could be directly connected or indirectly connected through intermediate media, or they could refer to communication within two components or the interaction between two components, unless otherwise clearly specified. A person skilled in the art can understand the specific meaning of these terms in the context of this application based on the situation.

In this application, unless otherwise clearly specified, when a first feature is "on" or "below" a second feature, it can mean that the first and second features are in direct contact or that the first and second features are in indirect contact via an intermediate medium. Furthermore, when a first feature is "above", "on top of", or "over" a second feature, it can mean the first feature is directly above or at an oblique angle above the second feature, or it can simply indicate that the first feature is at a higher horizontal height than the second feature. Similarly, when a first feature is "below", "under", or "beneath" a second feature, it can mean that the first feature is directly below or at an oblique angle below the second feature, or it can simply indicate that the first feature is at a lower horizontal height than the second feature.

It should be noted that when a component is referred to as being "fixed to" or "disposed on" another component, it can be directly on the other component or can exist with an intermediary component. When one component is said to be "connected" to another, it can be directly connected to the other component or may also involve intermediate components. The terms "vertical", "horizontal", "up", "down", "left", "right", and similar expressions used in this document are only for illustrative purposes and do not indicate the only possible implementation.

## Claims

1. A vascular stent, comprising:
a front stent section, a middle stent section and a rear stent section connected sequentially, an outer surface of the front stent section and/or the rear stent section being provided with an anchoring protrusion,
wherein when the vascular stent is expanded, an internal diameter of the middle stent section is smaller than internal diameters of the front stent section and the rear stent section, the front stent section is configured to contact an inner wall of a blood vessel, the rear stent section is configured to contact the inner wall of the blood vessel, and the anchoring protrusion is configured to anchor the vascular stent.

2. The vascular stent of claim **1,** wherein a proximal end and/or a distal end of the front stent section is provided with a radiopaque marker, and a proximal end and/or a distal end of the rear stent section is provided with a radiopaque marker.

3. The vascular stent of claim 1 or 2, wherein the front stent section, the middle stent section, the rear stent section, and the anchoring protrusion are integrally formed from a shape memory alloy material.

4. The vascular stent of any one of claims 1 to 3, wherein the front stent section, the middle stent section and the rear stent section are integrally formed from a shape memory alloy material to obtain a main body of the vascular stent, the anchoring protrusion is connected with the main body of the vascular stent by clamping, welding or bonding, and the anchoring protrusion is made of a degradable material.

5. The vascular stent of any one of claims 1 to 4, wherein the front stent section and/or the rear stent section includes a connecting portion provided with the anchoring protrusion.

6. The vascular stent of any one of claims 1 to 5, wherein an outer surface of the front stent section and/or the rear stent section carries a drug.

7. The vascular stent of any one of claims 1 to 6, wherein the front stent section, the middle stent section, and the rear stent section each have a mesh structure, shapes of mesh apertures of the front stent section, the middle stent section, and the rear stent section are each rectangular, rhombic, circular, or oval, and an area of the mesh aperture of the middle stent section gradually decreases from both ends of the middle stent section towards a middle region of the middle stent section.

8. The vascular stent of claim 7, wherein the front stent section, the middle stent section, and the rear stent section of the stent each include multiple unit frames forming the mesh apertures, each unit frame includes at least four connecting bars sequentially connected end to end and connecting portions each connected between adjacent connecting bars, and two adjacent unit frames share one connecting portion.

9. The vascular stent of any one of claims 1 to 8, wherein an outer surface and/or an inner surface of the middle stent section is provided with a covering.

10. The vascular stent of any one of claims 1 to 9, wherein a minimum inner diameter of the middle stent section in an expanded state is defined as D1, where D1 ranges from 1.5mm to 6mm, an inner diameter of the front stent section in an expanded state is defined as D2, where D2 ranges from 3mm to 18mm, an inner diameter of the rear stent section in an expanded state is defined as D3, where D3 ranges from 3mm to 18mm; and an inner diameter of the middle stent section gradually decreases from both two ends of the middle stent section the to a center of the middle stent section.

11. The vascular stent of any one of claims 1 to 10, wherein a length of the front stent section in an expanded state is defined as L1, and a length of the rear stent section in an expanded state is defined as L2, where L1 ranges from 0.2mm to 15mm, and L2 ranges from 0.2mm to 15mm.
